# EUROPÄISCHE PATENTSCHRIFT

(11) **EP 2 022 414 B1**
(45) Veröffentlichungstag und Bekanntmachung des Hinweises auf die Patenterteilung: **02.12.2009**
(21) Anmeldenummer: 08011923.3
(22) Anmeldetag: 02.07.2008
(51) Int. Cl.: A61B 17/16, B23B 51/04

(54) **Schädelbohrer**
Skull drill
Dispositif de forage de crâne

(30) Priorität: 31.07.2007 DE 102007036354
(43) Veröffentlichungstag der Anmeldung: 11.02.2009
(73) Patentinhaber: MEDICON EG CHIRURGIEMECHANIKER-GENOSSENSCHAFT, D-78532 Tuttlingen (DE)
(72) Erfinder: Burger, Andreas, 78532 Tuttlingen (DE); Schmid, Volker, 78532 Tuttlingen (DE); Wenzler, Klaus, 78665 Frittlingen (DE)
(74) Vertreter: Mammel, Ulrike

(56) Entgegenhaltungen:
- EP-A- 0 843 987
- US-A- 4 951 690

## Beschreibung

Die Erfindung betrifft einen Schädelbohrer, der aus einer Aufnahmevorrichtung für einen Bohrkopf und einem Bohrkopf besteht.

Solche Schädelbohrer, die bspw. aus der DE 38 90 886 C2, dem US 4,951,690 C2 und der DE 38 81 120 C2 bekannt sind, werden zum Setzen von Löchern in Schädelknochen in der Neurochirurgie eingesetzt. Im Rahmen einer Operation kann ein direkter Zugang zu dem kranialen Raum und dem Gehirn erhalten werden. Dies ist insbesondere bei Patienten mit der Creutzfeldt-Jakob-Krankheit erforderlich. Diese Schädelbohrer sind derart konstruiert, dass der Bohrkopf und die Aufnahmeeinheit für den Bohrkopf in einem unbetätigten Zustand bezüglich eines Drehantriebes entkoppelt sind. Bei Anbringen der Aufnahmevorrichtung an der Bohrstelle zum Einbringen einer Bohrung wird nach dem Aufsetzen der Aufnahmevorrichtung an der Schädeldecke der Bohrkopf mit der Aufnahmevorrichtung durch eine Axialkupplung verkuppelt, so dass eine Drehbewegung auf den Bohrkopf übertragen wird. Beim Durchbrechen der Schädeldecke erfolgt im Moment des Durchbruches ein Entkoppeln des Antriebs und eine Stillsetzung des Bohrkopfes, in dem die Axialkupplung ausgekuppelt wird. Dies erfolgt bspw. über einen Federdruck. Durch diese Sicherheitsfunktion ist ein Hauptbohrer des Bohrkopfes nur solange wirksam, wie seine vordere Spitze auf dem harten Knochen des Schädels arbeitet.

Beim Schädelbohrer gemäß der DE 38 90 886 C2 weist der Hauptbohrer des Bohrkopfes eine zentrale Gewindebohrung auf, welche an einem Gewindeabschnitt der Aufnahmeeinheit angreift, um eine dauerhafte Verbindung zu schaffen. Ein Auswechseln des Bohrkopfes als auch eine Reinigung des Schädelbohrers ist sehr zeitaufwendig, da ein Zerlegen des Schädelbohrers mit einer Vielzahl von Handgriffen erforderlich ist.

Beim Schädelbohrer gemäß der DE 38 81 120 C2 ist der Hauptbohrer des Bohrkopfes zur Aufnahmevorrichtung durch eine Stiftverbindung gehalten. Ein Schaft des Hauptbohrers weist eine Querbohrung auf, in der der Stift eingesetzt ist und eine Verbindung mit der Aufnahmevorrichtung schafft. Ein schnelles Auswechseln des Bohrkopfes sowie eine Reinigung eines solchen Schädelbohrers ist aufgrund der zur Zerlegung des Schädelkopfes erforderlichen Handgriffe zeitaufwendig.

Aus der EP 0 843 987 sind Schädelbohrer mit einer lösbaren Rastkupplung zwischen Aufnahmevorrichtung und Bohrkopf bekannt. Die Rastkupplung ist zwischen einem Aufnahmeabschnitt am Grundkörper und einem Zapfen des Bohrkopfs angeordnet und der Zapfen weist eine randoffene Vertiefung auf, die zur Aufnahme eines Rastelements dient. Nachteilig ist an diesem Schädelbohrer jedoch, dass bei Betätigung der Rastkupplung die Entkopplung des Antriebs und das Stillsetzen des Bohrkopfes nicht sichergestellt sind.

Der Erfindung liegt deshalb die Aufgabe zugrunde, einen Schädelbohrer mit einer verbesserten Sicherheit bereitzustellen.

Diese Aufgabe wird erfindungsgemäß durch die Merkmale des Anspruchs 1 gelöst. Weitere vorteilhafte Ausgestaltungen und Weiterbildungen der Erfindung sind in den weiteren Ansprüchen angegeben.

Durch die erfindungsgemäße Ausgestaltung eines Schädelbohrers, bei dem sich die randoffene Vertiefung am Zapfen des Bohrkopfes in axialer Richtung über eine Länge erstreckt, die zumindest einer Verschiebebewegung für ein Auskuppeln der zwischen der Aufnahmevorrichtung und dem Bohrkopf wirkenden Axialkupplung entspricht, wird die Sicherheitsfunktion des Schädelbohrers aufrechterhalten, der ein Auskuppeln des Hauptbohrers von dem Drehantrieb bzw. von der Aufnahmevorrichtung dann erfordert, sobald die Schädeldecke durchbohrt ist. Gleichzeitig bleibt der Bohrkopf in der Aufnahmevorrichtung durch die lösbare Rastkupplung gefangen, so dass sich die Aufnahmevorrichtung von dem Bohrkopf nicht löst.

Ein schnelles und einfaches Eingreifen des zumindest einen Rastelementes in die Vertiefung am Zapfen zur Verriegelung erfolgt durch eine zur Aufnahmebohrung des Aufnahmeabschnitts weisende randoffene Vertiefung am Zapfen des Bohrkopfes. Durch die randoffene umlaufende Vertiefung ist eine spezielle Ausrichtung des Bohrkopfes zum Einsatz in der Aufnahmevorrichtung nicht erforderlich.

Durch die lösbare Rastkupplung wird ein schnelles Lösen des Bohrkopfes von der Aufnahmevorrichtung ermöglicht. Gleichzeitig wird durch das schnelle Lösen eine einfache Handhabung des Schädelbohrers bzw. der Aufnahmevorrichtung und des Bohrkopfes für Reinigungszwecke und Desinfektionszwecke ermöglicht. Darüber hinaus können schnell und einfach verschiedene Größen und Durchmesser am Bohrkopf als auch Bohrköpfe mit verschiedenen Schneidgeometrien von der Aufnahmevorrichtung aufgenommen werden.

Durch die Anordnung der lösbaren Rastkupplung zwischen einem Aufnahmeabschnitt am Grundkörper der Aufnahmevorrichtung und einem Zapfen des Bohrkopfes, insbesondere des Hauptbohrers, kann der Bohrkopf durch eine einfache Steckverbindung zur Aufnahmevorrichtung gehalten und gekuppelt werden. Der prinzipielle Aufbau von solchen Bohrköpfen, welche einen zylindrischen Hauptbohrer und eine hülsenförmige Bohrkrone umfassen, die auf dem Hauptbohrer axial verschiebbar ist, können beibehalten werden. Es versteht sich, dass gemäß einer alternativen Ausführungsform auch vorgesehen sein kann, dass der Grundkörper der Aufnahmevorrichtung einen Zapfen aufweist und der Bohrkopf einen Aufnahmeabschnitt umfasst, um die Aufnahmevorrichtung und den Bohrkopf durch eine lösbare Steckrastverbindung miteinander zu verbinden.

Nach einer weiteren bevorzugten Ausgestaltung der Erfindung ist vorgesehen, dass die lösbare Rastkupplung zwischen der Aufnahmebohrung des Aufnahmeabschnitts und dem Zapfen am Bohrkopf zumindest ein Rastelement aufweist, welches aus einer Eingriffsposition in eine Freigabeposition überführbar ist. Durch eine solche Ausgestaltung kann eine einfache kraftschlüssige Verriegelung der beiden Kupplungselemente gegeben sein. Darüber hinaus kann eine innenliegende lösbare Rastkupplung geschaffen werden.

Bevorzugt ist vorgesehen, dass zum Lösen der lösbaren Rastkupplung eine Verriegelungshülse betätigbar ist, welche koaxial zum Grundkörper angeordnet ist und axial zum Aufnahmeabschnitt des Grundkörpers verschiebbar ist. Durch eine Schiebebewegung kann die Verriegelungshülse das zumindest eine Rastelement aus einer Eingriffsposition mit dem Zapfen des Bohrkopfes in eine Freigabeposition überführen. Somit ist eine einfache Handhabung und Lösen des Bohrkopfes von der Aufnahmevorrichtung ohne zusätzliche Werkzeuge ermöglicht.

Zwischen dem Grundkörper und der Verriegelungshülse der Aufnahmevorrichtung ist des Weiteren vorteilhafterweise ein Kraftspeicherelement vorgesehen, welches die Verriegelungshülse in einer Verriegelungsposition hält, in der das zumindest eine Rastelement in einer Eingriffsposition am Zapfen des Bohrkopfes gehalten ist. Dadurch ist eine selbsthaltende Verriegelung gegeben, die nur durch ein aktives Betätigen lösbar ist. Das Kraftspeicherelement ist bevorzugt als Druckfeder ausgebildet, welche innerhalb der Verriegelungshülse in einem mit dem Grundkörper gebildeten Aufnahmeraum vorgesehen ist.

Die lösbare Rastkupplung umfasst bevorzugt Rastelemente, welche zumindest in radialer Richtung verschiebbar zum Zapfen des Bohrkopfes geführt sind. Dadurch können einfache geometrische Verhältnisse geschaffen werden, um eine verschließbare Rastkupplung auszugestalten. Bevorzugt sind die Rastelemente als Rastkugeln oder als federgelagerte Stifte ausgebildet. Des Weitere können auch schwenkbare Klauen oder dergleichen eingesetzt werden. Der radialen Bewegungsrichtung kann auch eine axiale Bewegungsrichtung und/oder eine Schwenkbewegung überlagert sein.

Des Weiteren ist bevorzugt vorgesehen, dass im Grundkörper der Aufnahmevorrichtung dem Aufnahmeabschnitt gegenüberliegend ein Halteabschnitt vorgesehen ist, an dem ein Spannkegel zum Befestigen der Aufnahmevorrichtung an eine Antriebsvorrichtung vorgesehen ist. Dieser Spannkegel schließt den Halteabschnitt ab und ermöglicht mit seinem gegenüberliegenden Anschlussflächen eine einfache und schnelle Positionierung an der Antriebsvorrichtung. Bevorzugt ist der Spannkegel lösbar am Halteabschnitt vorgesehen, so dass im Schadensfall auch ein einfaches Zerlegen der Aufnahmevorrichtung ermöglicht ist.

Nach einer weiteren bevorzugten Ausgestaltung der Erfindung ist vorgesehen, dass im Grundkörper der Aufnahmevorrichtung dem Aufnahmeabschnitt gegenüberliegend ein Halteabschnitt vorgesehen ist, in dem eine Druckplatte geführt ist, welche mit einem mit Druckkraft beaufschlagbaren Kraftspeicherelement zur Aufnahmebohrung positioniert wird. Diese Druckplatte ist in dem Halteabschnitt axial verschiebbar durch das Kraftspeicherelement gehalten. Dieses Kraftspeicherelement ist bevorzugt als Druckfeder ausgebildet. Durch diese Druckplatte und dem Kraftspeicherelement ist die Ausdruckkraft der Axialkupplung und der Auslösezeitpunkt der Sicherheitsfunktionen bestimmt.

Diese Druckplatte greift bevorzugt an einer Stirnseite des Zapfen des Bohrkopfes an, der in einer Eingriffsposition zum Aufnahmeabschnitt angeordnet ist. Durch diese Druckplatte wird die Sicherheitsfunktion realisiert, indem in einem der Aufnahmevorrichtung durch die lösbare Rastkupplung gehaltener Bohrkopf in einer Auskuppelposition der Axialkupplung gehalten ist.

Des Weiteren ist bevorzugt vorgesehen, dass zwischen dem Aufnahmeabschnitt und dem Halteabschnitt am Grundkörper eine Schulter ausgebildet ist, an welcher die Druckplatte in einer Freigabeposition der Aufnahmevorrichtung anliegt. Dadurch wird eine kompakte und innenliegende Anordnung eines Teils der Axialkupplung ermöglicht. Gleichzeitig wird ein schnelles Auswechseln des Bohrkopfes zur Aufnahmevorrichtung sichergestellt.

Nach einer vorteilhaften Weiterbildung der Erfindung ist vorgesehen, dass zwischen der Aufnahmebohrung des Aufnahmeabschnittes und dem Zapfen des Bohrkopfes eine Dichtung vorgesehen ist. Bspw. kann eine O-Ring-Dichtung am Zapfen aufgebracht sein oder eine O-Ring-Dichtung in eine Nut der Aufnahmebohrung eingesetzt sein. Dadurch wird das Eindringen von Flüssigkeiten und Verschmutzungen in die Aufnahmevorrichtung während der Anwendung verhindert.

Nach einer weitern bevorzugten Ausgestaltung der Erfindung ist vorgesehen, dass in der Druckplatte ein Verschlussstück verschiebbar geführt ist, welches einen in den Aufnahmeabschnitt ragenden Ringbund aufweist, der das zumindest eine Rastelement in einer Freigabeposition der Aufnahmevorrichtung in dem Aufnahmeabschnitt sichert. Diese Anordnung ermöglicht insbesondere eine Trennung der Sicherheitsfunktion und der lösbaren Rastkupplung. Zum einen wird dadurch die lösbare Rastkupplung in einer Freigabeposition gehalten, welche zum schnellen Überführen in einer Eingriffsposition ohne zusätzliche Handhabung aus einem Aufstecken des Bohrkopfes auf die Aufnahmevorrichtung vorgesehen ist. Darüber hinaus werden die Rastelemente definiert und gesichert im Aufnahmeabschnitt positioniert.

Bevorzugt ist vorgesehen, dass in dem Aufnahmeabschnitt Bohrungen vorgesehen sind, in welchen die bevorzugt als Rastkugeln ausgebildeten Rastelemente eingesetzt sind, welche aus einer Freigabeposition und in einer Eingriffsposition überführbar sind. Dies ermöglicht einfache geo-metrische Ausgestaltungen und eine kompakte Bauweise.

Des Weiteren ist bevorzugt vorgesehen, dass in der zumindest einen Bohrung in dem Aufnahmeabschnitt zur Aufnahme des Rastelementes gegenüberliegend ein Einstich in der Verschiebehülse zuordenbar ist, sobald die Verschiebehülse in einer Verriegelungsposition angeordnet ist. Durch dieses zumindest teilweise Eingreifens des zumindest einen Rastelementes in den Einstich, wird die Verschiebehülse in einer Entriege-lungsposition fixiert. Dadurch wird nach dem Einsetzen des Bohrkopfes in die Aufnahmevorrichtung beim Erreichen der Eingriffsposition für das zumindest eine Rastelement ein selbständiges Verriegeln ermöglicht und die lösbare Rastkupplung greift. Dies erfolgt dadurch, dass das auf die Verschiebehülse wirkende Kraftspeicherelement eine axiale Verschiebebewegung der Verschiebehülse in eine Verriegelungsposition einwirkt und dadurch die Rastkugeln in die umlaufende Vertiefung des Zapfens gedrückt werden. Nach der Einnahme dieser Position ist die Verschiebehülse derart verschoben, dass die Bohrung in dem Aufnahmeabschnitt zumindest teilweise geschlossen ist. Dadurch wird gleichzeitig die Eingriffsposition der Rastelemente an dem Bohrkopf gesichert. Somit wird durch dies Anordnung eine einfache Handhabung sichergestellt.

Bevorzugt ist des Weiteren vorgesehen, dass die Wandstärke des Aufnahmeabschnitts kleiner als der Durchmesser einer als Rastelement ausgebildeten Rastkugel vorgesehen ist. Dadurch kann die Doppelfunktion in einfacher Weise verwirklicht werden, nämlich zum einen das Fixieren der Verriegelungshülse in einer Entriegelungsposition sobald die Aufnahmevorrichtung in eine Freigabeposition übergeführt ist und zum anderen das sichere Verriegeln der lösbaren Rastkupplung nach dem Überführen des Bohrkopfes in eine Eingriffsposition zur Aufnahmevorrichtung.

Des Weiteren ist bevorzugt vorgesehen, dass der Ringbund des Verschlussstückes an der Aufnahmebohrung geführt ist und in einer Freigabeposition eine äußere Ringfläche des Ringbundes die zumindest eine Bohrung überdeckt. Dadurch kann eine leicht gängige und eine leicht lösbare Rastkupplung geschaffen sein.

Des Weiteren ist bevorzugt vorgesehen, dass das Verschlussstück mit einem mit Druck beaufschlagbaren Kraftspeicherelement in dem Halteabschnitt angeordnet ist. Dieses zusätzliche Kraftspeicherelement, welches insbesondere als Druckfeder oder Spiralfeder ausgebildet ist, ermöglicht zum einen die Sicherung der Rastelemente im Aufnahmeabschnitt beim Überführen der Aufnahmevorrichtung in eine Freigabeposition und zum anderen weist dieses Kraftspeicherelement eine Auswerferfunktion auf, um den Zapfen des Bohrkopfes zumindest teilweise aus der Aufnahmebohrung des Aufnahmeabschnittes herauszuführen.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
- Figur 1: Eine schematische Seitenansicht eines Schädelbohrers,
- Figur 2: eine schematische Schnittdarstellung entlang der Linie I - I in Figur 1,
- Figur 3: eine schematische Ansicht von unten auf den Schädelbohrer gemäß Figur 1,
- Figur 4: eine schematische Schnittdarstellung des Schädelbohrers gemäß Figur 1 in einer Anordnung, bei welcher eine Aufnahmevorrichtung von einem Bohrkopf getrennt ist,
- Figur 5: eine schematische Seitenansicht einer alternativen Ausführungsform zu Figur 1,
- Figur 6: eine schematische Schnittdarstellung entlang der Linie V - V in Figur 5,
- Figur 7: eine schematische Seitenansicht des Schädelbohrers gemäß Figur 5 mit einem von einer Aufnahmevorrichtung gelösten Bohrkopf und
- Figur 8: eine schematische Schnittdarstellung entlang der Linie VII - VII in Figur 7.

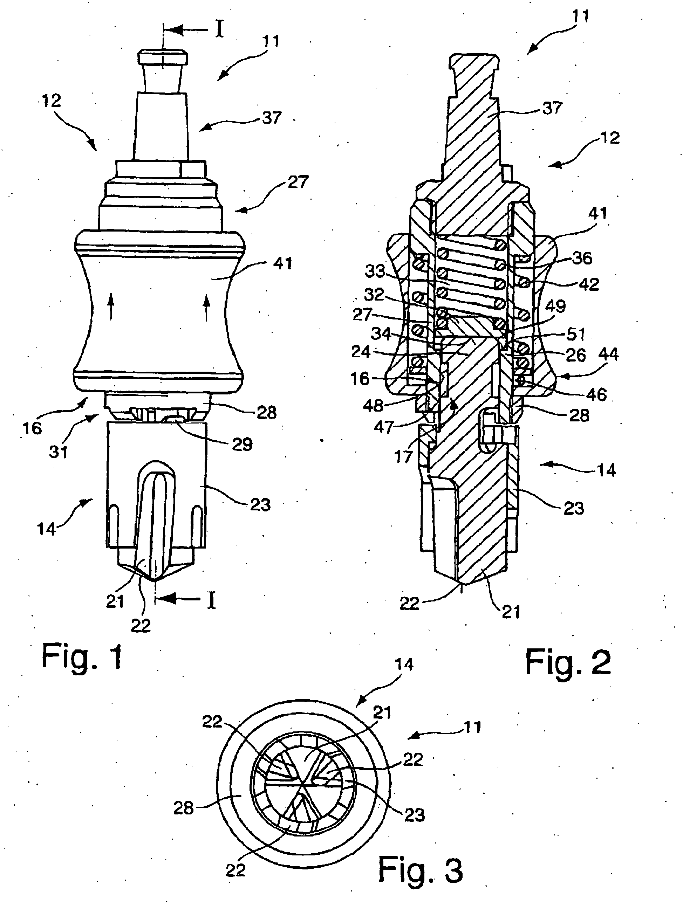

In Figur 1 ist eine schematische Seitenansicht eines erfindungsgemäßen Schädelbohrers 11 dargestellt. Die Figur 2 zeigt eine schematische Schnittansicht des Schädelbohrers 11 entlang der Linie I - I in Figur 1. Die Figur 3 zeigt eine Ansicht von unten auf den Schädelbohrer 11 gemäß Figur 1. Der Schädelbohrer 11 besteht aus einer Aufnahmevorrichtung 12 und einem Bohrkopf 14. Der Bohrkopf 14 ist gemäß den Figuren 1 und 2 in einer Eingriffsposition 16 durch eine lösbare Rastkupplung 17 gehalten. Durch diese lösbare Rastkupplung 17 ist der Bohrkopf 14 in einfacher Weise austauschbar zur Aufnahmevorrichtung 12 angeordnet.

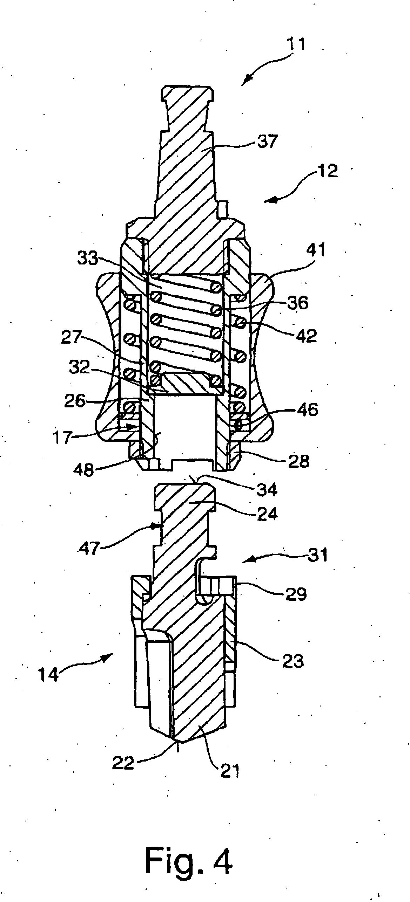

Der Bohrkopf 14 umfasst einen Hauptbohrer 21, welcher bspw. 3 Schneiden 22 umfasst. Dieser Hauptbohrer 21, der bevorzugt zylindrisch ausgebildet ist, wird von einer bevorzugt hülsenförmig ausgebildeten Bohrkrone 23 umgeben. Diese Bohrkrone 23 ist axial verschiebbar zum Hauptbohrer 21 angeordnet. Der Bohrer 21 umfasst einen Zapfen 24, der in einem Aufnahmeabschnitt 26 eines Grundkörpers 27 der Aufnahmevorrichtung 12 lösbar über die Rastkupplung 17 gehalten ist. An einer zum Bohrkopf 14 weisenden Stirnseite des Grundkörpers 27 ist eine Mitnehmerscheibe 28 vorgesehen, welche zusammen mit an dem Hauptbohrer 21 angeordneten Mitnehmern 29 eine Axialkupplung 31 bildet. Sobald der Bohrkopf 14 an einer Bohrsteile aufgesetzt wird, erfolgt eine Axialverschiebung des Hauptbohrers 21 in den Aufnahmeabschnitt 26 der Aufnahmevorrichtung 12, wobei die Axialkupplung 31 einrückt, indem die Mitnehmer 29 in die Mitnehmerscheibe 28 eingreifen. Dadurch kann eine Rotationsbewegung der Aufnahmevorrichtung 12 auf den Bohrkopf 14 übertragen werden. In der in Figur 4 dargestellten Position ist die Axialkupplung 31 ausgerückt, da eine Druckkraft über eine Druckplatte 32 in einem Halteabschnitt 33 des Grundkörpers 27 auf eine Stirnseite 34 des Zapfens 24 wirkt. Ein in dem Halteabschnitt 33 angeordnetes Kraftspeicherelement 36, welches bevorzugt als Druck- oder Spiralfeder ausgebildet ist, bestimmt die aufzubringende Axialkraft zum Einrücken oder Auskuppeln der Axialkupplung 31.

Der Halteabschnitt 33 ist dem Bohrkopf 14 gegenüberliegend mit einem Spannkegel 32 in dem sich das Kraftspeicherelement 36 ab. Der Spannkegel 37 dient zur Aufnahme in einem Bohrwerkzeug oder eine Antriebvorrichtung, welche nicht näher dargestellt ist.

Der Grundkörper 27 ist von einer Verriegelungshülse 41 umgeben, welche die lösbare Rastkupplung 17 in eine Eingriffsposition 16 gemäß Figur 2 oder in einer nachfolgenden Figur 4 beschriebenen Freigabeposition 56 anordnet. Die Verriegelungshülse 41 ist axial zum Grundkörper 27 verschiebbar angeordnet. Durch ein Kraftspeicherelement 42, welches sich mit einem Ende an einer Schulter des Grundkörpers 27 abstützt und am anderen Ende auf die Verriegelungshülse 41 einwirkt, wird die Verriegelungshülse 41 in einer Verriegelungsposition 44 gehalten. In dieser Verriegelungsposition 44 greift ein Rastelement 46 in eine ringförmige Vertiefung 47 in Zapfen 24 ein, so dass der Bohrkopf 14 zum Aufnahmeabschnitt 26 in einer Aufnahmebohrung 48 gehalten ist.

Das Rastelement 46 ist beispielsweise als federbelasteter Druckstift oder Druckstück ausgebildet, welches in radialer Richtung auslenkbar ist. Beispielsweise kann über eine Einführschräge 49 beim Einsetzen des Zapfens 24 in die Aufnahmebohrung 48 ein einfaches Überführen in eine Entriegelungsposition ermöglicht werden und nach dem vollständigen Positionieren des Zapfens 24 in dem Aufnahmeabschnitt 26 ein selbständiges Einrasten in der Vertiefung 47 gegeben sein. Zum Entriegeln der lösbaren Rastkupplung 17 wird die Verriegelungshülse 21 entgegen dem Kraftspeicherelement 42 bewegt, wobei das Rastelement 46 über eine am Aufnahmeabschnitt 26 angeordnete Formschräge in eine Entriegelungsposition übergeführt wird, so dass anschließend ein einfaches Herausführen des Zapfens 24 aus der Aufnahmebohrung 48 des Aufnahmeabschnitts 26 ermöglicht ist.

Zur Aufrechterhaltung der Sicherheitsfunktion und Betätigung der Axialkupplung 31 ist vorgesehen, dass die Vertiefung 47 zumindest eine solche Breite umfasst, dass ein vollständiges Ausrücken der Mitnehmer 29 aus der Mitnehmerscheibe 28 ermöglicht ist. Unabhängig von diesem Verschiebeweg bleibt das Rastelement 46 in einer Eingriffsposition zum Zapfen 24 des Bohrkopfes 14.

Die in Figur 4 dargestellte Schnittansicht des Schädelbohrers 11 zeigt den Bohrkopf 14 getrennt zur Aufnahmevorrichtung 12. In dieser Anordnung liegt die Druckplatte 32 an einer Schulter 51 am Grundkörper 27 an. Diese Schulter 51 ist im Übergangsbereich zwischen dem Aufnahmeabschnitt 26 und dem Halteabschnitt 33 gebildet. Der Bohrkopf 14 wird zum Befestigen an der Aufnahmevorrichtung 21 mittels dem Zapfen 24 in die Aufnahmebohrung 48 eingeführt und solange nach oben bewegt, bis die Formschräge 49 das zumindest eine Rastelement 46 in eine Entriegelungsposition überführt. Anschließend liegt die Stirnseite 34 des Zapfens 24 an der Druckplatte 32 an. Diese wird ebenfalls weiter entgegen dem Kraftspeicherelement 36 in den Halteabschnitt 33 hineinbewegt, bis das zumindest eine Rastelement 46 in die Vertiefung 37 eingreift. Somit ist eine einfache Montage des Bohrkopfes 14 zur Aufnahmevorrichtung 12 durch die lösbare Rastkupplung 17 ermöglicht.

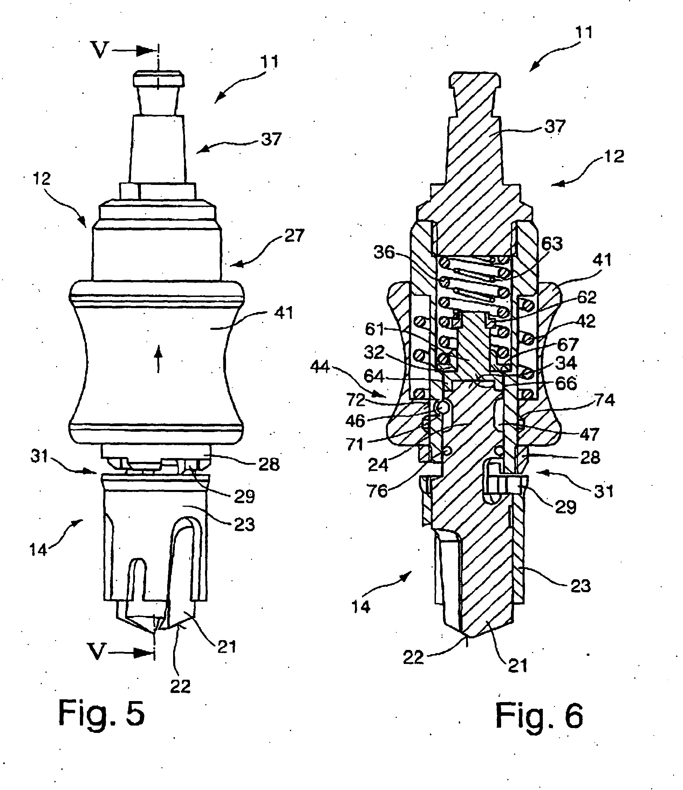

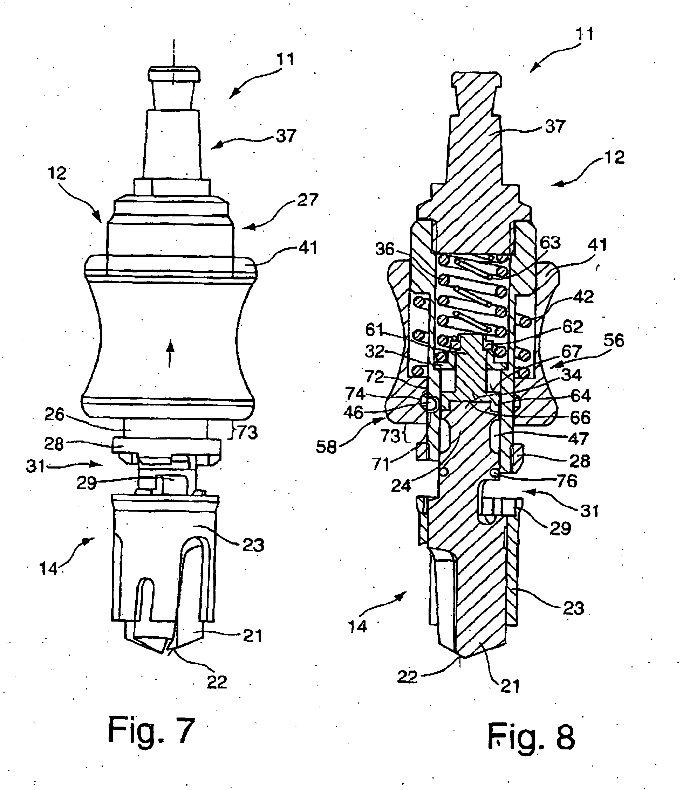

In den Figuren 5 bis 8 ist eine alternative Ausführungsform des Schädelbohrers 11 gemäß den Figuren 1 bis 4 dargestellt. Die Figuren 5 und 6 zeigen einen Schädelbohrer 11, bei dem der Bohrkopf 14 durch die lösbare Rastkupplung 17 in die Aufnahmevorrichtung 12 gehalten ist. Die Figuren 7 und 8 zeigen eine Anordnung des Bohrkopfes 14 zur Aufnahmevorrichtung 12, bei welcher die lösbare Rastkupplung 17 in einer Freigabeposition 56 angeordnet ist. Im Hinblick auf die mit den Figuren 1 bis 4 übereinstimmenden Merkmale und Vorteile wird auf die vorstehende Figurenbeschreibung Bezug genommen. Nachfolgend werden die zusätzlichen bzw. abgewandelten Merkmale näher beschrieben.

Dieser Schädelbohrer 11 weist wie aus Figur 6 hervorgeht ein Verschlussstück 61 auf, welches in der Druckplatte 32 geführt ist. Dieses Verschlussstück 61 weist an einem in den Halteabschnitt 33 ragenden Ende ein Halteelement 62 zur Aufnahme eines Kraftspeicherelementes 63 auf, welches sich gegenüberliegend an bspw. dem Spannkegel 37 oder einem Verschlusselement für den Halteabschnitt 33 abstützt. An einem in den Aufnahmeabschnitt 26 ragenden Ende des Verschlussstückes 61 ist ein Ringbund 64 vorgesehen, welcher in der Aufnahmebohrung 48 geführt ist. Dieser Ringbund 64 weist eine Stirnfläche 66 auf, an welcher die Stirnseite 34 des Zapfens 24 in einer Verriegelungsposition 44 der Aufnahmevorrichtung 12 anliegt. Um die Sicherheitsfunktion weiterhin zu gewährleisten, liegt eine Ringfläche 67 des Verschlussstückes 61 an der Druckplatte 32 an. Der Ringbund 64 ist somit lediglich zwischen der Stirnseite 34 des Zapfens 24 und der Druckplatte 32 zwischengeschalten.

Durch das zusätzliche Anordnen eines Verschlussstückes 61 mit dem Ringbund 64 wird zum einen eine Auswerferfunktion ermöglicht, um den Bohrkopf 14 einfach aus der Aufnahmebohrung 48 bzw. dem Aufnahmeabschnitt 26 zu lösen. Des Weiteren wird eine Anordnung der Verriegelungshülse 41 in eine Freigabeposition 56 der Aufnahmevorrichtung 12 ermöglicht, welche in Figur 7 dargestellt und in der Schnittdarstellung gemäß Figur 8 in der Funktion ersichtlich wird.

Die lösbare Rastkupplung 17 weist als Rastelement 46 Kugeln bzw. Rastkugeln auf, welche in einer Bohrung 71 im Aufnahmeabschnitt 26 vorgesehen sind. Vorzugsweise sind mehrere Rastelemente 46 gleichmäßig über den Umfang verteilt angeordnet. Es kann aber auch nur ein Rastelement 46 vorgesehen sein.

Bei einer Axialbewegung bzw. Verschiebebewegung der Verriegelungshülse 41 entgegen dem Kraftspeicherelement 42 in eine Entriegelungsposition 58 wird ein Verriegelungsabschnitt 72, der die Bohrung 71 in einer Eingriffsposition der Rastkupplung 17 überdeckt, wie dies in Figur 6 dargestellt ist, nach oben bewegt. Dieser Verschiebeweg ist durch die Bezugsziffer 73 dargestellt. Gleichzeitig wird dadurch ein Einstich 74 in der Verriegelungshülse 41 zur Bohrung 71 des Aufnahmeabschnitts 26 positioniert, wodurch ermöglicht wird, dass das Rastelement 46 derart radial nach außen wandert, dass der Ringbund 64 die Bohrung 71 überdeckt und schließt und das Rastelement 46 zumindest teilweise in den Einstich 74 eingreift. Bevorzugt kann an dem stirnseitigen freien Ende des Ringbundes 64 ebenfalls eine Formschräge vorgesehen sein, um das Rastelement 46 beim Überführen der Rastkupplung 17 aus einer Eingriffsposition 16 in eine Freigabeposition 56 radial nach außen zu bewegen. Dadurch ist die Verriegelungshülse 41 in einer Entriegelungsposition 58 und die Aufnahmevorrichtung 12 in eine Freigabeposition 56 übergeführt.

Das Verschlussstück 61 wird aufgrund des Halteelementes 62 in einer Endlage positioniert, bei dem der Ringbund 64 die Bohrung 71 schließt oder überdeckt. Dadurch wird das Rastelement 46 in dem Einstich 74 positioniert, wodurch wiederum erzielt wird, dass die Verriegelungshülse 41 in einer in Figur 8 dargestellten Entriegelungsposition 58 für den Bohrkopf 14 gehalten und verriegelt ist. Dies ermöglicht, dass die Aufnahmevorrichtung 12 in der Freigabeposition 56 vorgesehen ist.

Unmittelbar nach dem Einsetzen des Zapfens 24 in den Aufnahmeabschnitt 26 erfolgt ein selbständiges Überführen der lösbaren Rastkupplung 17 aus der Freigabeposition 56 in eine Eingriffsposition 16. Durch das Kraftspeicherelement 42 wirkt eine Schräge des Einstichs 74 der Verriegelungshülse 41 auf das Rastelement 46 ein, so dass dieses in die Bohrung 71 hineinbewegt wird, um auf der gegenüberliegenden Seite in die Vertiefung 47 des Zapfens 24 einzugreifen. Anschließend überdeckt der Verriegelungsabschnitt 72 der Verriegelungshülse 41die Bohrung 71. Die Verriegelungshülse 41 wird über einen Anschlag in dieser Endposition gehalten, der vorteilhafterweise durch die Mitnehmerscheibe 28 gebildet ist. Durch die Anordnung der Rastelemente 46 in der Vertiefung 47 kann der Hauptbohrer 21 axial verschiebbar in der Aufnahmevorrichtung 12 aufgenommen werden, um ein Ein- und Ausrücken der Axialkupplung 31 zu ermöglichen, ohne dass der Bohrkopf 14 sich von der Aufnahmevorrichtung 12 löst. Zum Ein- und Ausrücken der Axialkupplung 31 wirkt bei dieser Ausführungsform sowohl das Kraftspeicherelement 36 als auch das Kraftspeicherelement 63.

Bei dieser Ausführungsform gemäß den Figuren 5 bis 8 ist bspw. an dem Zapfen 24 eine Dichtung 76 vorgesehen, um das Eindringen von Flüssigkeit in den Aufnahmeabschnitt 26 zu verhindern. Zusätzlich kann auch zwischen der Verriegelungshülse 41 und dem Grundkörper 27 eine Verriegelungshülse 41 vorgesehen sein.

## Patentansprüche

1. Schädelbohrer mit einer Aufnahmevorrichtung (12) für einen Bohrkopf (14) und mit einem Bohrkopf (14), der einen zylindrischen Hauptbohrer (21) und eine hülsenförmige Bohrkrone (23) umfasst, die auf dem Hauptbohrer (21) axial verschiebbar ist, und mit einer zwischen dem Bohrkopf (14) und der Aufnahmevorrichtung (12) angeordneten Axialkupplung (31), welche beim Aufsetzen des Bohrkopfes (14) an einer Schädeldecke einrückbar ist, so dass eine Drehbewegung auf den Bohrkopf (14) übertragen wird und beim Durchbrechen der Schädeldecke entkuppelt wird, um den Bohrkopf (14) stillzusetzen, wobei die Aufnahmevorrichtung (12) und der Bohrkopf (14) durch eine lösbare Rastkupplung zueinander angeordnet sind und die lösbare Rastkupplung (17) zwischen einem Aufnahmeabschnitt (26) am Grundkörper (27) der Aufnahmevorrichtung (12) und einem Zapfen (24) des Hauptbohrers (21) des Bohrkopfes (14) angeordnet ist und der Zapfen (24) eine zur Aufnahmebohrung (48) des Aufnahmeabschnitts (26) weisende randoffene Vertiefung (47) umfasst,
**dadurch gekennzeichnet,**
**dass** die Vertiefung (47) im Zapfen (24) des Bohrkopfes (14) sich in axialer Richtung über eine Länge erstreckt, welche zumindest einem Verschiebeweg für ein Auskuppeln der zwischen der Aufnahmevorrichtung (12) und dem Bohrkopf (14) wirkenden Axialkupplung (31) entspricht.

2. Schädelbohrer nach Anspruch 1, **dadurch gekennzeichnet, dass** die lösbare Rastkupplung (17) zwischen dem Aufnahmeabschnitt (26) der Aufnahmevorrichtung (12) und dem Zapfen (24) am Bohrkopf (14) zumindest ein Rastelement (46) umfasst, welches aus einer Eingriffsposition (16) in eine Freigabeposition (56) überführbar ist.

3. Schädelbohrer nach einem der vorhergehenden Ansprüche, **dadurch gekennzeichnet, dass** die Aufnahmevorrichtung (12) eine koaxial zum Grundkörper (21) angeordnete Verriegelungshülse (41) aufweist, welche axial zum Aufnahmeabschnitt (26) verschiebbar ist und an dem zumindest einen Rastelement (46) angreift.

4. Schädelbohrer nach Anspruch 3, **dadurch gekennzeichnet, dass** zwischen dem Grundkörper (27) und der Verriegelungshülse (41) ein Kraftspeicherelement (42) vorgesehen ist, durch welche die Verriegelungshülse (41) in einer Verriegelungsposition (44) vorgesehen ist, in der das zumindest eine Rastelement (46) in einer Eingriffsposition (16) am Zapfen (24) des Bohrkopfes (14) gehalten ist.

5. Schädelbohrer nach Anspruch 1, **dadurch gekennzeichnet, dass** die lösbare Rastkupplung (17) zumindest ein Rastelement (46) aufweist, welches zumindest in radialer Richtung verschiebbar zum Zapfen (24) des Bohrkopfes (14) oder zum Aufnahmeabschnitt (26) der Aufnahmevorrichtung (12) vorgesehen ist.

6. Schädelbohrer nach Anspruch 1, **dadurch gekennzeichnet, dass** der Grundkörper (27) der Aufnahmevorrichtung (12) einen dem Aufnahmeabschnitt (26) gegenüberliegenden Halteabschnitt (33) aufweist, an dem ein Spannkegel (37) zum Befestigen der Aufnahmevorrichtung (12) an einer Antriebsvorrichtung vorgesehen ist.

7. Schädelbohrer nach Anspruch 1, **dadurch gekennzeichnet, dass** am Grundkörper (27) der Aufnahmevorrichtung (12) dem Aufnahmeabschnitt (26) gegenüberliegend ein Halteabschnitt (33) vorgesehen ist, in dem eine Druckplatte (32) verschiebbar aufgenommen ist, welche mit einem Kraftspeicherelement (36) in Richtung zum Aufnahmeabschnitt (26) mit einer Druckkraft beaufschlagbar ist.

8. Schädelbohrer nach Anspruch 7, **dadurch gekennzeichnet, dass** die in dem Halteabschnitt (33) angeordnete Druckplatte (32) an einer Stirnseite (34) des Zapfens (24) des Bohrkopfes (14) in einer Eingriffsposition (16) der Rastkupplung (17) angreift.

9. Schädelbohrer nach Anspruch 7, **dadurch gekennzeichnet, dass** zwischen dem Aufnahmeabschnitt (26) und dem Halteabschnitt (33) am Grundkörper (27) eine Schulter (51) ausgebildet ist, an welcher die Druckplatte (32) in einer Freigabeposition (56) der Aufnahmevorrichtung (12) anliegt.

10. Schädelbohrer nach Anspruch 1, **dadurch gekennzeichnet, dass** zwischen der Aufnahmebohrung (48) und dem Zapfen (24) des Bohrkopfes (14) eine Dichtung (76) angeordnet ist.

11. Schädelbohrer nach Anspruch 7, **dadurch gekennzeichnet, dass** in der Druckplatte (32) ein Verschlussstück (61) verschiebbar geführt ist, welches einen in den Aufnahmeabschnitt (26) ragenden Ringbund (64) aufweist, der das zumindest eine Rastelement (46) in eine Freigabeposition (56) der Aufnahmevorrichtung (12) sichert.

12. Schädelbohrer nach Anspruch 11, **dadurch gekennzeichnet, dass** in dem Aufnahmeabschnitt (26) für das als Rastkugel ausgebildete zumindest eine Rastelement (46) eine Bohrung (71) vorgesehen ist, in der das Rastelement (26) zwischen einer Freigabeposition (56) und einer Eingriffsposition (16) verschiebbar angeordnet ist.

13. Schädelbohrer nach Anspruch 12, **dadurch gekennzeichnet, dass** an einer Außenseite des Aufnahmeabschnittes (26) der Bohrung (71) zugeordnet ein Einstich (74) der Regelungshülse (41) zuordenbar ist, in welche das zumindest eine Rastelement (46) eingreift, sobald die Verriegelungshülse (41) in eine Entriegelungsposition (58) übergeführt ist.

14. Schädelbohrer nach Anspruch 13, **dadurch gekennzeichnet, dass** der Aufnahmeabschnitt (26) eine Wandstärke aufweist, die kleiner als ein Durchmesser eines als Rastkugel ausgebildeten Rastelementes (46) ist.

15. Schädelbohrer nach Anspruch 11, **dadurch gekennzeichnet, dass** der Ringbund (64) des Verschlussstückes (61) in der Aufnahmebohrung (48) des Aufnahmeabschnitts (26) geführt ist und in einer Freigabeposition (46) eine äußere Ringfläche des Ringbundes (64) die zumindest eine Bohrung (71) im Aufnahmeabschnitt (26) zumindest teilweise überdeckt.

16. Schädelbohrer nach Anspruch 11, **dadurch gekennzeichnet, dass** das Verschlussstück (61) mit einem mit Druck beaufschlagbaren Kraftspeicherelement (63) in eine Freigabeposition (46) überführbar ist.

## Claims

1. A skull drill having a retaining device (12) for a drill head (14) and a drill head (14) comprising a cylindrical main drill (21) and a sleeve-like drill bit (23) which is axially displaceable on the main drill (21) as well as an axial coupling (31), arranged between the drill head (14) and the retaining device (12), which is engageable as the drill head (14) is applied on a skull cap such that a rotary movement is transmitted onto the drill head (14) and which becomes disengaged as the drill head transpierces the skull cap in order to stop said drill head (14), the retaining device (12) and the drill head (14) being arranged with respect to each other by means of a releasable snap-in coupling and said releasable snap-in coupling (17) being arranged between a retaining portion (26) on the main body (27) of the retaining.device (12) and a tenon (24) of the main drill (21) of the drill head (14), and said tenon (24) comprising a recess (47) with an open rim facing towards the retaining bore (48) of said retaining portion (26),
**characterised in that**
the recess (47) in the tenon (24) of the drill head (14) extends axially over a length corresponding at least to one displacement path required for releasing the axial coupling (31) acting between the retaining device (12) and the drill head (14).

2. The skull drill of claim 1, **characterised in that** the releasable snap-in coupling (17) between the retaining portion (26) of the retaining device (12) and the tenon (24) on the drill head (14) comprises at least one snap-in member (46) capable of being transferred from an engagement position (16) to a release position (56).

3. The skull drill of any of the preceding claims, **characterised in that** the retaining device (12) has a locking sleeve (41) arranged coaxially to the main body (21) and which is capable of being axially displaced with respect to the retaining portion (26) and abuts on the at least one snap-in member (46).

4. The skull drill of claim 3, **characterised in that** between the main body (27) and the locking sleeve (41) an energy storing member (42) is provided by means of which the locking sleeve (41) is provided in a locking position (44) in which the at least one snap-in member (46) is maintained in an engagement position (16) on the tenon (24) of the drill head (14).

5. The skull drill of claim 1, **characterised in that** the releasable snap-in coupling (17) has at least one snap-in member (46) capable of being displaced at least in a radial direction with respect to the tenon (24) of the drill head (14) or with respect to the retaining portion (26) of the retaining device (12).

6. The skull drill of claim 1, **characterised in that** the main body (27) of the retaining device (12) has a holding portion (33) which is situated opposite the retaining portion (26) and on which a clamping cone (37) for fastening the retaining device (12) onto a driving apparatus is provided.

7. The skull drill of claim 1, **characterised in that** on the main body (27) of the retaining device (12) opposite the retaining portion (26) a holding portion (33) is provided in which a pressure plate (32) is slidably received and on which a compressive force issuing from an energy storing member (36) and directed towards the retaining portion (26) may be applied.

8. The skull drill of claim 7, **characterised in that** the pressure plate (32) arranged in the holding portion (33) abuts on an end face (34) of the tenon (24) of the drill head (14) when the snap-in coupling (17) is in an engagement position (16).

9. The skull drill of claim 7, **characterised in that** between the retaining portion (26) and the holding portion (33) on the main body (27) a shoulder (51) is formed on which the pressure plate (32) abuts when the retaining device (12) is in a release position (56).

10. The skull drill of claim 1, **characterised in that** between the retaining bore (48) and the tenon (24) of the drill head (14) a sealing (76) is arranged.

11. The skull drill of claim 7, **characterised in that** in the pressure plate (32) a closure piece (61) is provided so as to slide in a guided manner, said closure piece having an annular collar (64) which protrudes into the retaining portion (26) thus locking the at least one snap-in member (46) in a release position (56) of the retaining device (12).

12. The skull drill of claim 11, **characterised in that** in the retaining portion (26) for the at least one snap-in member (46), which is shaped in the form of a snap-in ball, a bore (71) is provided in which the snap-in member (46) is displaceable between a release position (56) and an engagement position (16).

13. The skull drill of claim 12, **characterised in that** on an outer surface of the retaining portion (26) associated with the bore (71) a recess (74) of the adjustment sleeve (41) may be associated with which the at least one snap-in member (46) engages when the locking sleeve (41) has been transferred to an unlocking position (58).

14. The skull drill of claim 13, **characterised in that** the retaining portion (26) has a wall thickness that is smaller than a diameter of a snap-in member (46) realised in the form of a snap-in ball.

15. The skull drill of claim 11, **characterised in that** the annular collar (64) of the closure piece (61) is guided within the retaining bore (48) of the retaining portion (26) and that in a release position (46) an outer, annular surface of the annular collar (64) at least partially overlaps the at least one bore (71) in the retaining portion (26).

16. The skull drill of claim 11, **characterised in that** the closure piece (61) may be transferred to a release position (46) by means of an energy storing member (63) on which pressure may be applied.

## Revendications

1. Dispositif de forage de crâne avec un dispositif de réception (12) pour une tête de forage (14) et avec une tête de forage (14) qui comprend un foret principal (21) cylindrique et une couronne de forage (23) en forme de manchon capable de se déplacer axialement sur le foret principal (21), et avec un dispositif d'accouplement axial (31), disposé entre la tête de forage (14) et le dispositif de réception (12), lequel peut être enclenché lors de la mise en place de la tête de forage (14) sur une calotte crânienne de telle sorte qu'un mouvement rotatif est transmis à la tête de forage (14) et est désaccouplé au moment même où la calotte crânienne est transpercée afin d'arrêter la tête de forage (14), le dispositif de réception (12) et la tête de forage (14) étant disposés l'un par rapport à l'autre par un dispositif d'accouplement par encliquetage séparable, et le dispositif d'accouplement par encliquetage (17) séparable étant disposé entre une partie réceptrice (26) du corps de base (27) du dispositif de réception (12) et un tenon (24) du foret principal (21) de la tête de forage (14), et le tenon (24) comprenant un creux (47) ouvert vers le bord orienté vers le trou de réception (48) de la partie de réception (26),
**caractérisé en ce que**
le creux (47) ménagé dans le tenon (24) de la tête de forage (14) s'étend en direction axiale sur une longueur qui correspond au moins à une distance de déplacement permettant un désaccouplage du dispositif d'accouplement axial (31) agissant entre le dispositif de réception (12) et la tête de forage (14).

2. Dispositif de forage de crâne selon la revendication 1, **caractérisé en ce que** le dispositif d'accouplement par encliquetage (17) séparable comprend, entre la partie de réception (26) du dispositif de réception (12) et le tenon (24) situé sur la tête de forage (14), au moins un élément d'arrêt (46), lequel peut passer d'une position d'engrènement (16) à une position de libération (56).

3. Dispositif de forage de crâne selon l'une des revendications précédentes, **caractérisé en ce que** le dispositif de réception (12) présente une douille de verrouillage (41) disposée coaxialement au corps de base (21), laquelle peut se déplacer axialement par rapport à la partie de réception (26) et s'appuie sur ledit au moins un élément d'arrêt (46).

4. Dispositif de forage de crâne selon la revendication 3, **caractérisé en ce que**, entre le corps de base (27) et la douille de verrouillage (41), est prévu un élément d'accumulation d'énergie (42) grâce auquel la douille de verrouillage (41) est mise en position de verrouillage (44), dans laquelle ledit au moins un élément d'arrêt (46) est maintenu dans une position d'engrènement (16) située sur le tenon (24) de la tête de forage (14).

5. Dispositif de forage de crâne selon la revendication 1, **caractérisé en ce que** le dispositif d'accouplement par encliquetage (17) séparable présente au moins un élément d'arrêt (46), lequel est prévu pour pouvoir se déplacer au moins en direction radiale par rapport au tenon (24) de la tête de forage (14) ou par rapport à la partie de réception (26) du dispositif de réception (12).

6. Dispositif de forage de crâne selon la revendication 1, **caractérisé en ce que** le corps de base (27) du dispositif de réception (12) présente, à l'opposé de la partie de réception (26), une partie de retenue (33) sur laquelle est prévu un cône de serrage (37) permettant de fixer le dispositif de réception (12) à un dispositif d'entraînement.

7. Dispositif de forage de crâne selon la revendication 1, **caractérisé en ce que**, sur le corps de base (27) du dispositif de réception (12), est prévue une partie de retenue (33) située à l'opposé de la partie de réception (26), dans laquelle est logée, de manière à pouvoir se déplacer, une plaque de compression (32) qui, grâce à un élément d'accumulation d'énergie (36), peut être soumise à une force de pression en direction de la partie de réception (26).

8. Dispositif de forage de crâne selon la revendication 7, **caractérisé en ce que** la plaque de compression (32) disposée dans la partie de retenue (33) s'appuie sur une face frontale (34) du tenon (24) de la tête de forage (14) lorsque le dispositif d'accouplement par encliquetage (17) se trouve dans une position d'engrènement (16).

9. Dispositif de forage de crâne selon la revendication 7, **caractérisé en ce que**, entre la partie de réception (26) et la partie de retenue (33), est formé, dans le corps de base (27), un épaulement (51) sur lequel repose la plaque de compression (32) lorsque le dispositif de réception (12) se trouve dans une position de libération (56).

10. Dispositif de forage de crâne selon la position 1, **caractérisé en ce qu'**un joint d'étanchéité (76) est disposé entre le trou de réception (48) et le tenon (24) de la tête de forage (14).

11. Dispositif de forage de crâne selon la revendication 7, **caractérisé en ce que** dans la plaque de compression (32) est disposé, de sorte à se déplacer de manière guidée, un élément obturateur (61) qui présente un collet annulaire (64) faisant saillie dans la partie de réception (26), lequel bloque ledit au moins un élément d'arrêt (46) lorsque le dispositif de réception (12) se trouve dans une position de libération (56).

12. Dispositif de forage de crâne selon la revendication 11, **caractérisé en ce que**, dans la partie de réception (26), est prévu, pour ledit au moins un élément d'arrêt (46) réalisé sous forme de sphère d'arrêt, un trou (71), dans lequel l'élément d'arrêt (46) est disposé de manière à pouvoir se déplacer entre une position de libération (56) et une position d'engrènement (16).

13. Dispositif de forage de crâne selon la revendication 12, **caractérisé en ce que**, sur une face extérieure de la partie de réception (26) attribuée au trou (71) peut être attribuée une entaille (74) de la douille de réglage (41), dans laquelle engrène ledit au moins un élément d'arrêt (46) dès que la douille de verrouillage (41) est passée dans une position de déverrouillage (58).

14. Dispositif de forage de crâne selon la revendication 13, **caractérisé en ce que** la partie de réception (26) présente une épaisseur de paroi qui est inférieure à un diamètre d'un élément d'arrêt (46) réalisé sous forme de sphère d'arrêt.

15. Dispositif de forage de crâne selon la revendication 11, **caractérisé en ce que** le collet annulaire (64) de l'élément obturateur (61) est guidé dans le trou de réception (48) de la partie de réception (26) et **en ce que**, dans une position de libération (46), une surface annulaire extérieure du collet annulaire (64) recouvre au moins partiellement ledit au moins un trou (71) ménagé dans la partie de réception (26).

16. Dispositif de forage de crâne selon la revendication 11, **caractérisé en ce que** l'élément obturateur (61) peut passer dans une position de libération (46) grâce à un élément d'accumulation d'énergie (63) pouvant être mis sous pression.
